Europäisches Patentamt

European Patent Office

Office européen des brevets

(19)

(11) Veröffentlichungsnummer : **0 173 267 B1**

(12) **EUROPÄISCHE PATENTSCHRIFT**

(45) Veröffentlichungstag der Patentschrift :
**17.05.89**

(51) Int. Cl.⁴ : **A 61 B 17/56**, A 61 B 17/16,
B 23 B 49/02

(21) Anmeldenummer : **85110641.9**

(22) Anmeldetag : **20.11.81**

(60) Veröffentlichungsnummer der früheren Anmeldung
nach Art. 76 EPÜ : **0053999**

(54) **Bohrlehre zum Bohren von schiefwinkelig zur Knochenlängsachse verlaufenden Platten-Schrauben-Löchern in zu stabilisierenden Knochenteilen.**

(30) Priorität : **20.11.80 CH 8599/80**

(43) Veröffentlichungstag der Anmeldung :
**05.03.86 Patentblatt 86/10**

(45) Bekanntmachung des Hinweises auf die Patenterteilung : **17.05.89 Patentblatt 89/20**

(84) Benannte Vertragsstaaten :
**BE CH DE FR LI SE**

(56) Entgegenhaltungen :
**FR–A– 2 233 972**
**FR–A– 2 242 068**
**FR–A– 2 246 343**
**FR–A– 2 348 686**
**FR–A– 2 480 106**
**US–A– 2 181 746**
**US–A– 2 697 433**

(73) Patentinhaber : **Synthes AG Chur**
**Grabenstrasse 15**
**CH-7002 Chur (CH)**

(72) Erfinder : **Klaue, Kaj**
**Spiegelstrasse 64**
**CH-3028 Spiegel-Bern (CH)**

(74) Vertreter : **Klingseisen, Franz, Dipl.-Ing. et al**
**Dr. F. Zumstein sen. Dr. E. Assmann Dr. F. Zumstein jun. Dipl.-Ing. F. Klingseisen Bräuhausstrasse 4**
**D-8000 München 2 (DE)**

EP 0 173 267 B1

**Beschreibung**

Die Erfindung betrifft eine Bohrlehre nach dem Oberbegriff des Anspruchs 1.

Der Stand der Technik der Kompressions-Osteosynthese, sowohl vorrichtungsmässig als auch operationstechnisch, ist zusammenfassend in der folgenden Veröffentlichung beschrieben :

a) M. E. Müller, M. Allgöwer, R. Schneider und H. Willenegger, Manual der Osteosynthese (AO-Technik), 2. Auflage, Springer-Verlag Berlin Heidelberg New York 1977

b) B. Claudi u. a., Hel. chir. Acta 46 (1979), 177-182.

Weiter ist bezüglich Vorrichtungen auf die folgenden Veröffentlichungen zu verweisen :

c) CH-PS 462 373
d) CH-PS 468 824
e) CH-PS 600 862
f) CH-PS 611 147
g) CH-PS 613 616
h) CH-PS 613 858.

Bis jetzt wurde bei der Kompressions-Osteosynthese die Kompressionsplatte überwiegend nach dem Zuggurtungsprinzip angewendet. Dabei wird die Platte einseitig auf den zu stabilisierenden Bereich an die auf Zug beanspruchte Seite des Knochens angebracht und nach Vorspannung mittels Schrauben lotrecht zur Knochen- und Plattenlängsachse mit dem Knochen verschraubt ; die der Platte gegenüberliegende Muskulatur sowie die physiologische Belastung schliesst durch zusätzliche dynamische Kompression den Frakturspalt (vgl. Veröffentlichung a)).

Statische, interfragmentäre Kompression kann durch einfache Zugschrauben ohne Verwendung einer Platte erreicht werden (Veröffentlichung a)).

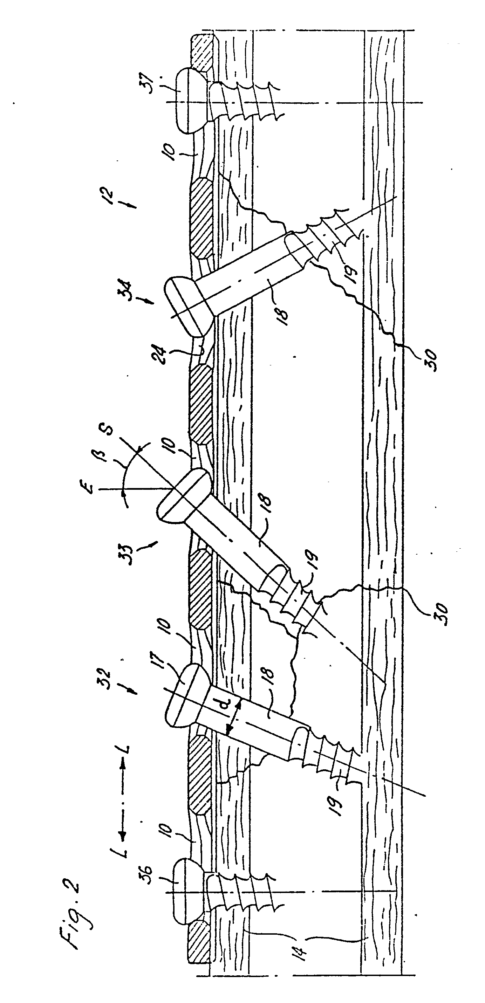

Neuerdings wurde auch bereits vorgeschlagen, die Stabilisierung mittels Platte durch Hinzufügen einer schiefwinklig zur Knochen- und Plattenlängsachse verlaufenden, die Frakturspaltebene durchdringenden schrägen Zugschaube, deren Axialkraft eine zusätzliche interfragmentäre Kompression bewirkt, zu verbessern (vgl. Veröffentlichung b), Seite 178 und Abb. 2B).

Der Anwendung der letzterwähnten Methode waren bisher jedoch enge Grenzen gesetzt, da die Geometrie der bisher bekannten Kompressionsplatten und Schrauben nur eine ungenügende Schwenkung der Schrauben gegenüber der Querebene und in geschwenktem und voll eingeschraubtem Zustande praktisch keine Längsbewegung der Schrauben, welche über ihre ganze Länge mit Gewinde versehen waren, in den Widerlagern der Löcher der Kompressionsplatte zuliess.

In der EP-A-81 810 467.1 ist eine verbesserte osteosynthetische Vorrichtung angegeben, mittels der Zugschrauben bis zu einem Winkel von 45° gegenüber einer Querebene zur Lochlängsachse schräg eingesetzt werden können.

Der Erfindung liegt die Aufgabe zugrunde, eine Bohrlehre für die Herstellung einer solchen Vorrichtung vorzuschlagen.

Diese Aufgabe wird durch die Merkmale im Kennzeichen des Anspruchs 1 gelöst.

Aus der FR-A-2 348 686 ist eine Bohrlehre zum Bohren von quer zur Knochenlängsachse verlaufenden Löchern in zu stabilisierenden Knochenteilen bekannt, wobei auf eine auf der Knochenoberfläche aufliegende Platte ein etwa zylindrischer Körper der Bohrlehre aufgesetzt wird, der eine senkrecht zur Knochenlängsachse verlaufende Bohrung zur Führung des Bohrers aufweist. Dabei verläuft die Achse dieser Bohrung etwa parallel zur Mittelachse des zylindrischen Körpers der Bohrlehre. Mit dieser bekannten Bohrlehre ist es nicht möglich, schiefwinkelig zur Knochenlängsachse verlaufende Löcher in zu stabilisierenden Knochenteilen zu bohren.

Vorteilhafte Ausgestaltungen der Erfindung sind in den weiteren Ansprüchen angegeben.

Beispielsweise Ausführungsformen der Erfindung sowie der Stand der Technik werden nachstehend anhand der Zeichnung näher erläutert. Es stellen dar :

Fig. 0 einen Längsschnitt durch eine mittels einer dynamischen Kompressionsplatte gemäss dem Stand der Technik fixierten Fraktur ;

Fig. 1 eine schematische Darstellung der bei Anwendung des Prinzips der schrägen Kompressionsschraube nach EP-A-81 810 467.1 entstehenden Kräfte und Momente ;

Fig. 2 einen Längsschnitt durch eine Ausführungsform der Vorrichtung in appliziertem Zustand an einer mehrfragmentären Fraktur ;

Fig. 3 einen Querschnitt durch eine Ausführungsform der Vorrichtung, bei welcher der Rand des Loches auf der vom Knochen abgewandten Seite der Platte gegenüber dem das Widerlager bildenden Teil der Wandungen nach aussen abgeschrägt ist ;

Fig. 4 einen Längsschnitt durch eine Ausführungsform der Vorrichtung während der Herstellung durch Fräsen eines Loches mittels eines Kugelfräsers ;

Fig. 5 einen Längsschnitt durch eine Ausführungsform der Vorrichtung während des Ausfräsens einer trichterförmigen Erweiterung ;

Fig. 6 einen Längsschnitt durch eine erste Ausführungsform einer erfindungsgemäßen Bohrlehre ;

Fig. 7 einen Längsschnitt durch eine Vorrichtung mit der in Fig. 6 dargestellten Bohrlehre in Applikationslage ;

Fig. 8 und 9 eine zweite Ausführungsform einer erfindungsgemäßen Bohrlehre in Längs- bzw. Querschnitt ; und

Fig. 10 die zeichnerische Darstellung der Zusammenhänge zwischen den Abmessungen der Bohrlehre, der Löcher und der Schrauben.

In Fig. 0 ist ein Längsschnitt durch eine mittels einer dynamischen Kompressionsplatte fixierte Fraktur dargestellt. Diese Darstellung entspricht dem Stand der Technik. Die Kompressionsplatte 1 liegt mindestens angenähert bündig auf dem Knochen 5 auf, weist mindestens zwei Löcher 3 auf (nur eins dargestellt) und ist dort mittels einer Kugelkopfschraube 2 am Knochen 5 fixiert. Letztere stösst am Lochrand 4 an, was beim Einschrauben zum Abdrücken des Kopfes der Kugelkopfschraube 2, d. h. Verschiebung der Schraube in Richtung der Platten- und Knochenlängsachse und des darunterliegenden Knochens, in Frakturrichtung führt. Das Fixieren der Kompressionsplatte 1 bewirkt die Entstehung von entgegengesetzt gerichteten Kräfte A und B an der Bruchstelle 6, 7. Dabei ist die Kraft B, welche die Bruchstelle 7 an der der Kompressionsplatte 1 abgewandten Seite auseinanderzieht, unerwünscht. Es wurde schon versucht, durch verschiedene Massnahmen der Kraft B entgegenzuwirken oder sie zu eliminieren, beispielsweise durch Biegen der Kompressionsplatte 1 (Literatur b)).

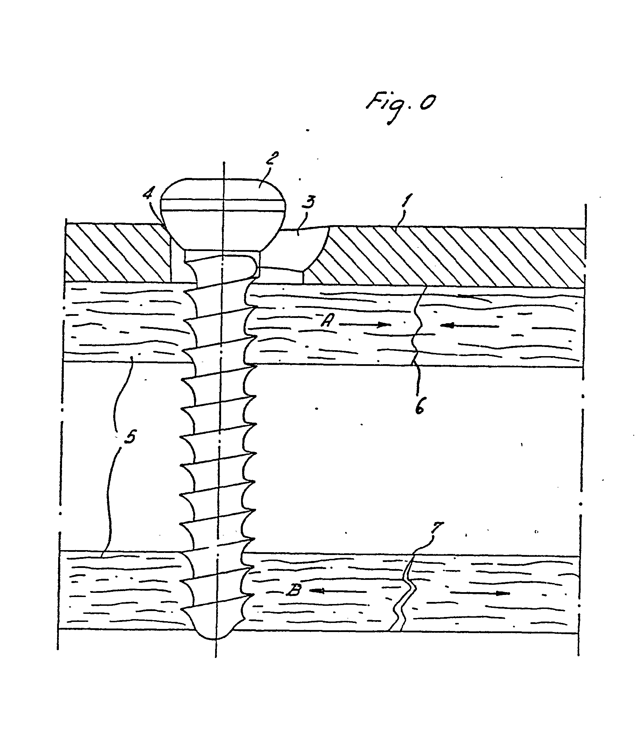

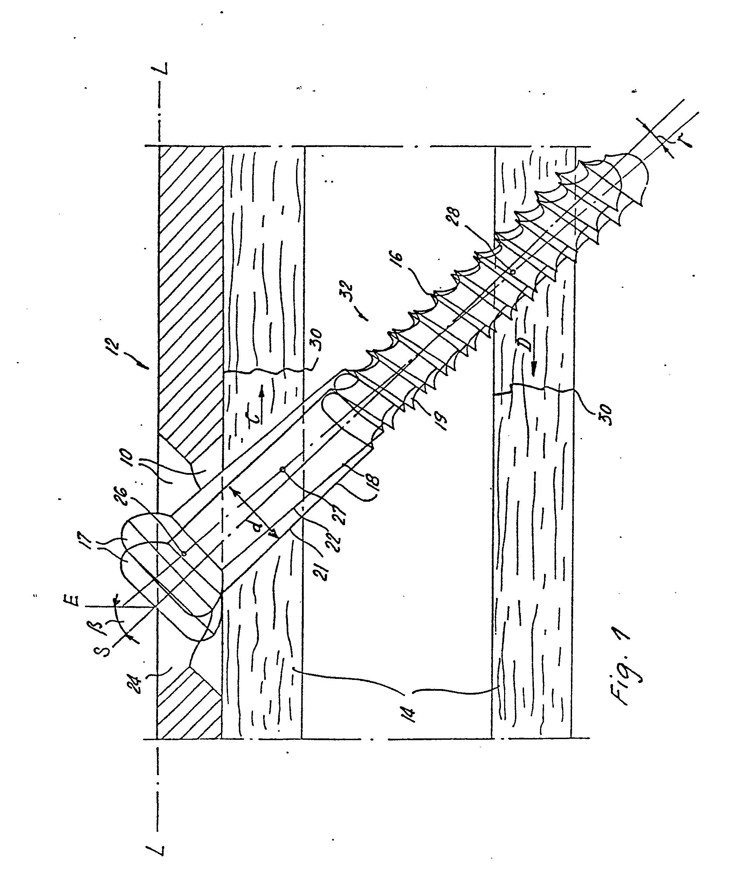

Fig. 1 zeigt dagegen die ebenfalls entgegengesetzt gerichteten Kräfte C, D, die bei Anwendung der Vorrichtung nach EP-A-81 810 467.1 entstehen. Die mit mehr als einem Loch 10 (nur eins dargestellt) versehene Platte 12 liegt wieder mindestens angenähert bündig auf dem Knochen 14 auf. Die Schraube 16 weist zwischen Schraubenkopf 17 und Gewindeteil 19 einen gewindefreien Teil 18 auf dessen Durchmesser d mindestens ebenso groß ist wie der Außendurchmesser des Gewindeteils 19, und ist schräg in den Knochen 14 eingesetzt, und zwar derart, dass sie mit einer lotrecht auf die Lochlängsachse stehenden Ebene E einen Neigungswinkel β von 45° bildet. Die Schraube 16 ist in zwei Stellungen 21 und 22 dargestellt, welchletztere sie durch Gleiten in der Gleitbahn 24 einnehmen kann. Durch das Gleiten der Schraube 16 entsteht eine Hebelwirkung, durch welche die Schraubenachse um den Winkel γ gekippt wird. Die Hebelwirkung ist derart, wie wenn die Kräfte in den Punkten 26, 28 angreifen würden und 27 der Stützpunkt des Hebels wäre. Auf die Bruchstelle 30 des Knochens 14 wirken die Kräfte C, D, welche im Gegensatz zur Fig. 0 die Bruchstücke an allen Stellen zusammendrücken.

In Fig. 2 wird ein Längsschnitt durch eine Ausführungsform der Vorrichtung in appliziertem Zustand an einer mehrfragmentären Fraktur 30 dargestellt. Die mit mehreren Löchern 10 versehene Platte 12 liegt wieder mindestens angenähert bündig auf dem Knochen 14 auf, welcher mehrere Frakturen 30 aufweist.

Die drei Schrauben 32, 33, 34 weisen zwischen Schraubenkopf 17 und Gewinde 19 einen gewindefreien Teil 18 auf. Die Schrauben 32, 33, 34 sind schräg eingesetzt, und zwar derart, dass sie mit einer lotrecht auf die Lochlängsachse stehenden Ebene E einen Neigungswinkel β bilden, welcher für die Schrauben 32, 33, 34 verschieden gross und vom Verlauf der Bruchstelle 30 abhängig ist. Die Schrauben 32, 33, 34 können in der jeweiligen Gleitbahn 24 gleiten. Bei den Schrauben 36, 37 handelt es sich um Plattenfixationsschrauben, deren Achsen lotrecht zur Lochlängsachse stehen. Sie dienen nur zur Befestigung der Platte 12 am Knochen 14. Währenddem die Anzahl der schräg eingesetzten Schrauben 32, 33, 34 von Art und Anzahl der Bruchstellen abhängig ist, werden vorzugsweise mindestens zwei Plattenfixationsschrauben, vorzugsweise an den beiden Längsenden der Platte 12, eingesetzt.

Die Schrauben können entweder mit einem solchen Gewinde versehen sein, welches das Vorschneiden der Gewinde im Bohrloch erforderlich macht oder auch selbstschneidende Gewinde aufweisen, welche is das gewindefreie Bohrloch eingeschraubt werden. Es können auch Schrauben verwendet werden, die über die ganze Länge des Schaftes mit Gewinde versehen sind.

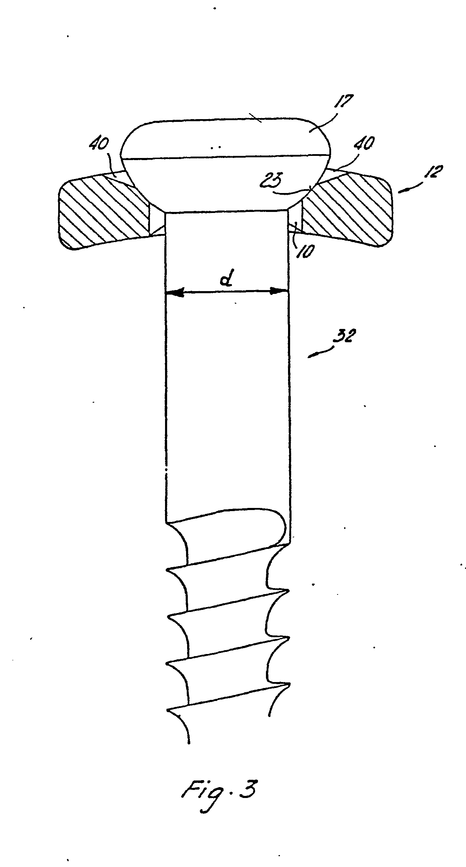

Fig. 3 ist ein Querschnitt durch eine Ausführungsform der Vorrichtung, bei welcher der Rand des Loches 10 auf der vom Knochen abgewandten Seite der Platte 12 gegenüber dem das Widerlager 23 bildenden Teil der Wandungen durch eine Abschrägung 40 nach außen versetzt ist.

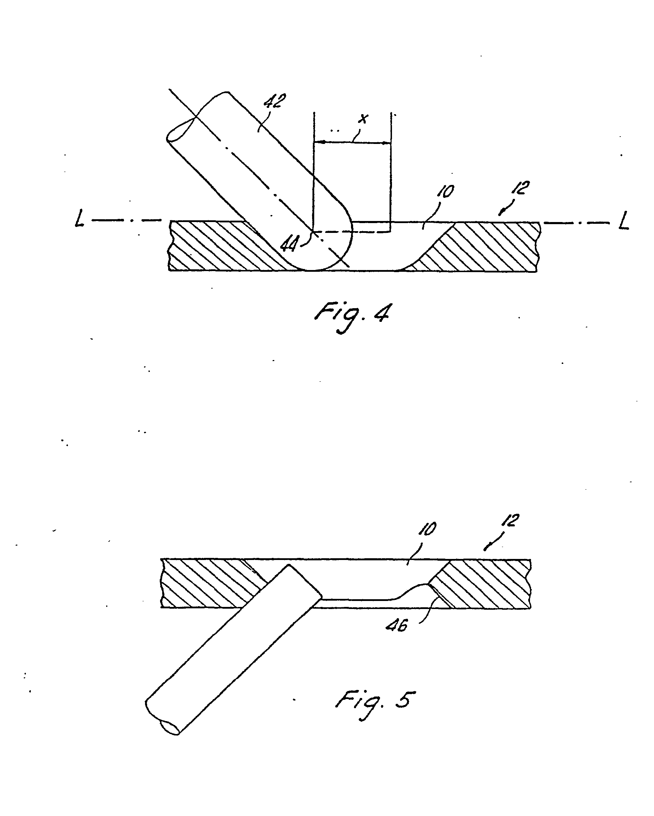

Fig. 4 zeigt einen Längsschnitt durch eine Ausführungsform der Vorrichtung während der Herstellung durch Fräsen eines Loches 10 mit einem Kugelfräser 42. Der Mittelpunkt 44 des Kopfes des Kugelfräsers 42 bewegt sich während des Fräsvorganges entlang der Strecke X. Vorteilhaft werden diese Löcher durch computergesteuerte Kugelfräser hergestellt.

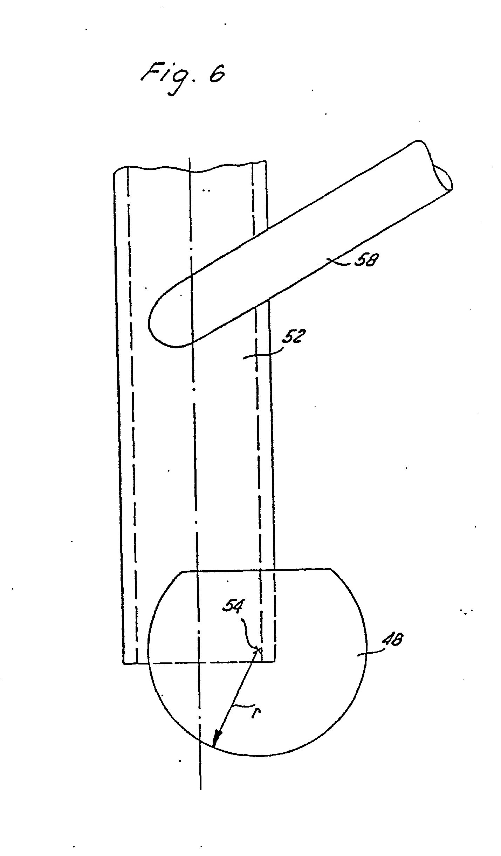

In Fig. 5 ist ein Längsschnitt durch eine Ausführungsform der Vorrichtung dargestellt. Es handelt sich um das Ausfräsen der trichterförmigen Erweiterung 46 des Loches 10 auf der dem Knochen zugewandten Seite der Platte 12.

Die Länge des Loches ist in Verbindung mit dem Schraubenschaft so ausgelegt, daß die Schraube unter einem Neigungswinkel β von bis zu 45° gegenüber der lotrecht auf der Lochlängsachse L stehenden Ebene E um mindestens eine Drittel des Schaftdurchmessers d, gemessen lotrecht zur Schraubenlängsachse S, gleiten kann (Fig. 1, 2).

Das durch die längsseitigen Wandungen des Loches 10 gebildete Widerlager 23 kann mindestens teilweise durch den Mantel eines Zylinders definiert sein, dessen Achse zur Lochlängsachse L parallel verläuft (Fig. 3, 4). Es ist aber auch möglich, daß das Widerlager 23 durch eine Ebene definiert wird, die sich mit der Abschrägung 40 in Fig. 3 in einer zur Lochlängsachse L parallelen Gerade schneidet.

Die Längsachse L des Loches 10 oder die Längsachsen mindestens eines Teils der Löcher können parallel, schiefwinkelig oder auch rechtwinkelig zur Längsachse der Platte verlaufen.

Die Ränder der Löcher auf der am Knochen anliegenden Seite der Platte 12 werden mindestens auf der frakturnahen Stirnseite des Loches 10 durch eine Abschrägung gegenüber der lichten Lochweite nach außen versetzt, wie dies z. B. Fig. 1 zeigt, so daß die Schraube auch nahe des Lochendes schräg eingesetzt werden kann, ohne mit dem Schaft am Lochrand anzuliegen. Eine solche stirnseitige Abschrägung kann auch auf der vom Knochen abgewandten Seite der Platte 12 an der frakturfernen Stirnseite des Loches vorgesehen werden.

Die längsseitigen Wandungen des Loches 10 sind als Gleitbahn 24 ausgebildet, in welcher der Schraubenkopf sich parallel zur jeweiligen Schraubenlängsachse S verschieben kann, wobei das Loch 10 und die Schraube 16 bzw. 32 bis 37 so ausgebildet, sind, daß die Schraube unter einem Neigungswinkel $\beta$ von bis zu 45° eingebracht werden und im Loch in dieser Stellung gleiten kann.

Die Ränder der Löcher 10 sind mindestens teilweise auf der vom Knochen abgewandten Seite der Platte 12 gegenüber dem das Widerlager 23 bildenden Teil der Wandungen durch eine Abschrägung bzw. eine abgeschrägte Fläche 40 nach außen versetzt. Durch diese Fläche 40 werden die beim Herstellungsvorgang auftretenden Kanten gebrochen, so daß beim Biegen und Verwinden der Platte die dazu benötigten Instrumente die Gleitbahn nicht beschädigen können. In entsprechender Weise sind die Ränder der Löcher 10 auf der am Knochen anliegenden Seite der Platte 12 gegenüber der lichten Lochweite über mindestens einen Teil des Lochumfanges durch eine abgeschrägte Fläche 46 nach außen versetzt.

Die Lochränder sind auf der am Knochen anliegenden Seite der Platte 12 mindestens auf der frakturnahen Stirnseite des Loches 10 und/oder auf der vom Knochen abgewandten Seite der Platte 12 an der frakturfernen Stirnseite des Loches gegenüber des lichten Loches durch eine Abschrägung nach außen versetzt.

Die Vorrichtung weist gegenüber dem Stand der Technik verschiedene Vorteile auf.

Wie in Fig. 0 gezeigt, wird die transkortikale Frakturspalte mit einer Vorrichtung gemäss dem Stand der Technik auseinandergezogen. Durch die schräge Lage der Schraube in der Vorrichtung wird eine doppelte Kompression möglich, da durch die Hebelarmwirkung (Fig. 1) nicht nur wie gemäss dem Stand der Technik die ciskortikale Frakturspalte sondern auch die transkortikale Frakturspalte zusammengedrückt wird. Entsprechend sind bei der Verwendung der Vorrichtung keine zusätzlichen Massnahmen, wie Biegen der Platte, zum Schliessen der transkortikalen Frakturspalte erforderlich.

Beim Anbringen einer Kompressionsplatte gemäss dem Stand der Technik ist der Spannweg, d. h. die Länge der Strecke, entlang welcher die Schraube samt dem von ihr gefassten Knochenteil in Richtung Fraktur geschoben wird, schon im voraus festgelegt. Demgegenüber kann die Kompression mit der Vorrichtung während des Einbringens der Schraube, infolge der Gleitmöglichkeit in der Gleitbahn, den vorherrschenden Umständen angepasst werden, da nicht die ganze mögliche Lochlänge ausgenutzt werden muss.

Der Kopf der Schraube der Vorrichtung wird bei der Verwendung nicht plastisch abgeknickt, d. h. der am Kopf der Schraube anschliessende Teil des Schraubenschaftes wird infolge Kippen der Schraubenachse um den Winkel $\gamma$ nicht in wesentlichem Ausmasse plastisch verbogen.

Bei Fixierung der Frakturen mittels einer Platte sollte letztere möglichst schmal und dünn sein, da unter der Platte eine Auflösung der Kortikalis, sogenannte Spongiosierung, stattfindet. Dieses Phänomen wird auch als Stressprotektion bezeichnet, wobei vermutet wird, dass es infolge Schutz des darunterliegenden Knochenbereiches gegen äussere Beanspruchung zustande kommt. Die Platte der Vorrichtung kann sehr dünn und schmal gestaltet werden, da infolge der transfragmentären Lage der Schraube die mechanische Belastung der Platte geringer wird. Bei Vorrichtungen gemäss dem Stand der Technik entsprach die Toleranz bezüglich der exzentrischen Schraubenlage im Plattenloch der Plattendicke. Da die Schraube in Richtung der Plattenachse und in geschwenkter Lage gleitbar und schwenkbar ist, kann eine fast beliebig dünne Platte eingesetzt werden, weil die Dickentoleranz der Platte nicht ausgenutzt werden muss.

Obwohl in den Figuren nur Röhrenknochen dargestellt sind, kann die Vorrichtung selbstverständlich auch zur Stabilisierung des Bruchbereiches in anderen Knochen eingesetzt werden.

Fig. 6 zeigt einen Längsschnitt durch eine erfindungsgemäße Bohrlehre, bei welcher das Lager 48 kugelförmig ausgebildet ist. Dieses weist den Radius r auf und ist bezüglich des Kugelmittelpunktes 54 exzentrisch mit der Bohrbüchse 52 verbunden. Letztere weist einen als Halterung dienenden Griff 58 auf.

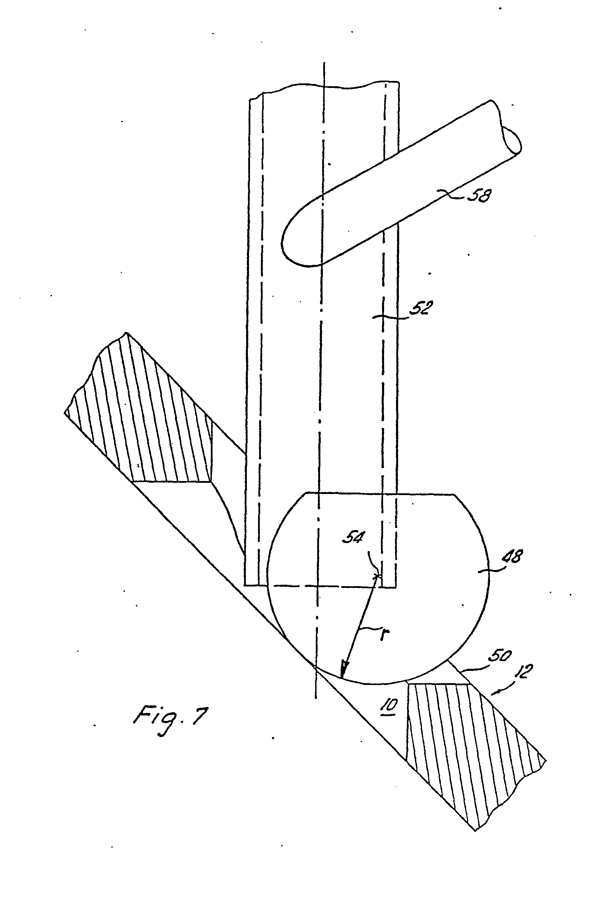

Fig. 7 zeigt einen Längsschnitt durch die Platte 12 der Vorrichtung mit der anglegten Bohrlehre nach Fig. 6. Das kugelförmige Lager 48 mit dem Radius r liegt in der Gleitbahn am frakturnahen Ende 50 des Loches 10. Die zur Führung des Bohrers bestimmte Bohrbüchse 52 ist bezüglich des Kugelmittelpunktes 54 exzentrisch mit dem kugelförmigen Lager 48 verbunden. Der Griff 58 ist an der Bohrbüchse 52 befestigt und dient als Halterung für die Bohrlehre.

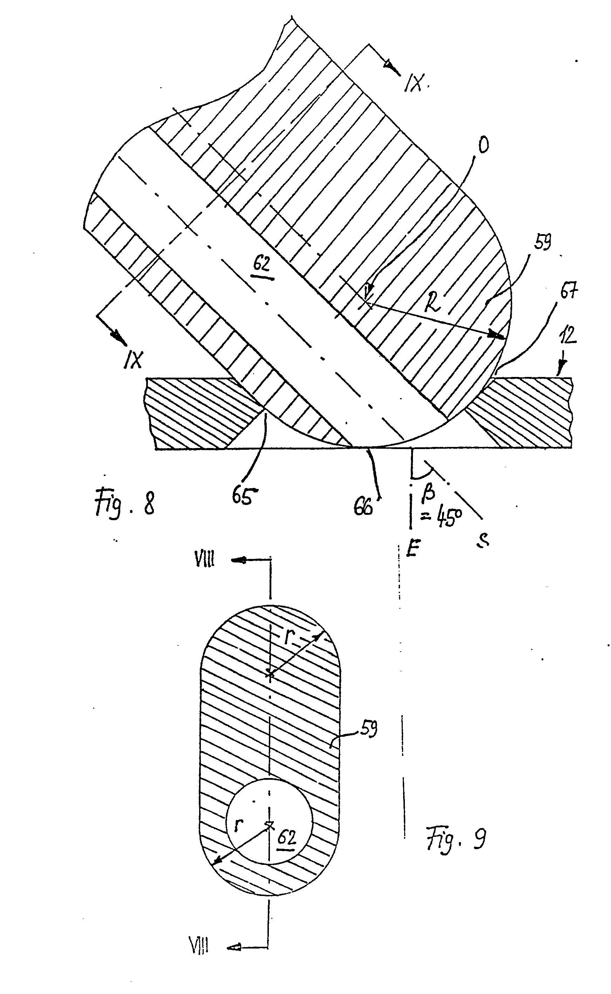

Fig. 8 und 9 zeigen eine zweite Ausführungsform einer erfindungsgemäßen Bohrlehre, und zwar Fig. 8 einem Längsschnitt entlang der Linie VIII-VIII in Fig. 9 und in Verbindung mit einer erfindungsgemässen Platte 12, Fig. 9 für sich allein einem Querschnitt nach der Linie IX-IX in Fig. 8.

Diese Bohrlehre weist ein kalottenartiges Lager 59 auf, in welches die Bohrbüchse 62 zur Führung des Bohrers exzentrisch eingelassen ist. Der in einer Schnittebene quer zur Lochlängsachse L liegende Radius r mit dem Mittelpunkt 0 des kalottenartigen Lagers 59 (Fig. 9) ist gleich dem Kugelradius der Kugelköpfe der Schrauben, während der in einer die Lochlängsachse L mitumfassenden Schnittebene VIII-VIII liegende Radius R des kalottenartigen Lagers 59 (Fig. 8) grösser als der Kugelradius der Kugelköpfe der Schrauben ist.

Die kongruenten Kontaktstellen zwischen Plattenloch 10 und Bohrlehre sind mit 65, 66 und 67 bezeichnet. Mit zunehmendem Radius R liegt schliesslich das kalottenartige Lager 59 auf der Geleitbahn 24 nicht mehr auf, sondern nur noch an den stirnseitigen Lochenden, so dass die Kontaktstelle 66 verschwindet und nur noch eine Zweipunktlagerung an den Kontaktstellen 65 und 67 stattfindet.

Durch diese Ausgestaltung der Bohrlehre wird zunächst einmal ihre optimale Zentrierung im Plattenloch 10 und in Richtung der Lochlängsachse L erreicht.

Bei einem Neigungswinkel β der Schraube bzw. des Bohrers von weniger als 45°, gemessen zwischen E und S, kommt der Schraubenkopf 17 beim Eindrehen der Schraube 16 auf die durch die längsseitigen Wandungen des Plattenloches 10 gebildete Gleitbahn 24, jedoch nie auf die schmalseitige Plattenkante zu liegen (Fig. 1). Dadurch wird eine besonders wirksame Kompression erreicht.

Bei einem Neigungswinkel β von 45° kommt der Schraubenkopf 17 beim Eindrehen der Schraube 16 direkt auf den Anfang der Gleitbahn 24 zu liegen. Die Kraft, die zwischen Schraube 16 und Platte 12 dabei übertragen wird, ist bei diesem extremen Winkelverhältnis am grössten und dürfte jedenfalls für eine wirksame Kompression genügen.

Bei senkrechter Stellung, d. h. bei einem Neigungswinkel β von 0°, kann die Bohrlehre auch als normale exzentrische Bohrlehre für die zum Stand der Technik gehörende Kompressions-Osteosynthese nach Fig. 0 eingesetzt werden.

Der Fuss der Bohrlehre kann mit Arretierungen versehen sein (in der Zeichnung nicht dargestellt), welche die Festlegung eines bestimmten Neigungswinkels β zwischen 0 und 45° erleichtert und auch eine leichte Seitenschwenkung ermöglichen.

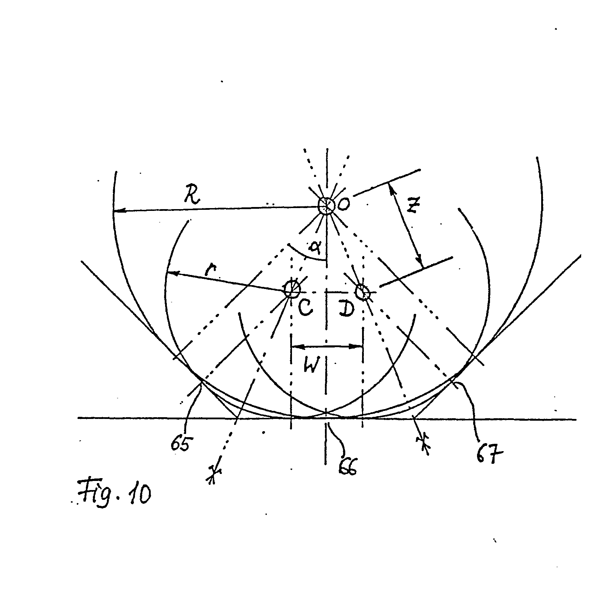

Die der Ausführungsform der erfindungsgemäßen Bohrlehre nach Fig. 8 und 9 zugrundeliegenden Zusammenhänge zwischen den beiden Radien r und R des kalottenartigen Lagers, dem parallel zur Lochlängachse gemessenen Gleitweg W der Schraube im Loch und dem Neigungswinkel α der Gleitbahn sind aus Fig. 10 ersichtlich.

Die kongruenten Kontaktstellen sind gleich wie in Fig. 8 mit 65, 66 und 67 bezeichnet. Die Zentren der Schraubenköpfe bei Extremlagen der Schrauben auf der Gleitbahn 24 sind mit C und D bezeichnet. Der Abstand der Punkte C und D entspricht dem horizontalen Gleitweg W.

Dabei gelten die Beziehungen:

$$R = r + \left[ \cos\frac{\alpha}{2} \cdot z \right] \tag{1}$$

und

$$z = \frac{W}{2} \cdot \frac{1}{\sin\frac{\alpha}{2}} \tag{2}$$

Daraus ergibt sich der Zusammenhang zwischen den oben erwähnten Grössen:

$$R = r + \left[ \frac{W}{2} \cdot ctg\frac{\alpha}{2} \right]$$

**Patentansprüche**

1. Bohrlehre zum Bohren von schiefwinklig zur Knochenlängsachse verlaufenden Löchern in zu stabilisierenden Knochenteilen mittels einer Vorrichtung aus einer Kompressionsplatte (12) mit darin länglich ausgebildeten Löchern (10) und dazu passenden Kugelkopfschrauben (16 ; 32-37) zur Stabilisierung des Bereichs eines Knochenbruches oder einer Osteotomie bei der Kompressions-Osteosynthese, dadurch gekennzeichnet, daß sie eine zur Führung des Bohrers bestimmte, in einem kalottenartigen Lager (48, 59) angeordnete Bohrbüchse (52, 62) aufweist, wobei die Achse der Bohrbüchse (52, 62) exzentrisch zum Mittelpunkt (54, 0) des Lagers (48, 59) liegt und das kalottenartige Lager (48, 59) dazu bestimmt ist, beim Bohren in den Löchern (10) der Platte (12) zu ruhen, wobei mindestens der in einer Schnittebene quer zur Längsachse (L) der Löcher (10) liegende Radius (r) des kalottenartigen Lagers (48, 59) gleich dem Kugelradius der Kugelköpfe (17) der Schrauben (16 ; 32-37) ist (Fig. 7-10).

2. Bohrlehre nach Anspruch 1, dadurch gekennzeichnet, dass das kalottenartige Lager Kugelform (48) aufweist und dazu bestimmt ist, beim Bohren in der Gleitbahn (24) der Löcher (10) der Platte (12) am frakturnahen Ende des Loches (10), wobei sein Radius (r) gleich dem Kugelradius der Kugelköpfe (17) der Schrauben (16 ; 32-37) ist (Fig. 7).

3. Bohrlehre nach Anspruch 1, dadurch gekennzeichnet, dass der in einer Schnittebene (IX-IX) quer zur Lochlängsachse (L) liegende Radius (r) des kalottenartigen Lagers (59) gleich dem Kugelradius der Kugelköpfe (17) der Schrauben (16 ; 32-37) ist, während der in einer die Lochlängsachse (L) mitumfassenden Schnittebene (VIII-VIII) liegende Radius (R) grösser als der Kugelradius der Kugelköpfe (17) der Schrauben (16 ; 32-37) ist (Fig. 8, 9).

4. Bohrlehre nach Ansprüche 1 und 3, dadurch gekennzeichnet, dass die beiden Radien (r, R) des kalottenartigen Lagers (59), der Gleitweg (W) der Schraube (16) im Loch (10) und der Neigungswinkel ($\alpha$) der Gleitbahn (24) durch die folgende Gleichung miteinander verknüpft sind :

$$R = r + \left[ \frac{W}{2} \cdot \text{ctg} \frac{\alpha}{2} \right].$$

## Claims

1. Drill guide for drilling holes, inclined relative to the longitudinal axis of the bone, in bone parts to be stabilized by means of a device comprising a compression plate (12) with holes (10) designed oblong therein and spherical head screws (16 ; 32-37) fitting in these for stabilizing the area of a bone fracture or an osteotomy in compression osteosynthesis, characterized in that it has a defined drill bushing (52, 62), for guiding the drill, arranged in a calotte-shaped bearing (48, 59), in which respect the axis of the drill bushing (52, 62) lies eccentric to the midpoint (54, 0) of the bearing (48, 59), and the calotte-shaped bearing (48, 59) is intended to rest, during drilling, in the holes (10) of the plate (12), and in which respect at least the radius (r), lying in a cutting plane transverse to the longitudinal axis (L) of the holes (10), of the calotte-shaped bearing (48, 59) is equal to the spherical radius of the spherical heads (17) of the screws (16 ; 32-37) (Figs. 7-10).

2. Drill guide according to Claim 1, characterized in that the calotte-shaped bearing has a spherical form (48) and is intended (sic), during drilling, in the guideway (24) of the holes (10) of the plate (12) at the fracture-near end of the hole (10), in which respect its radius (r) is equal to the spherical radius of the spherical heads (17) of the screws (16 ; 32-37) (Fig. 7).

3. Drill guide according to Claim 1, characterized in that the radius (r), lying in a cutting plane (IX-IX) transverse to the longitudinal axis (L) of the hole, of the calotte-shaped bearing (59) is equal to the spherical radius of the spherical heads (17) of the screws (16 ; 32-37), while the radius (R), lying in a cutting plane (VIII-VIII) including the longitudinal axis (L) of the hole, is greater than the spherical radius of the spherical heads (17) of the screws (16 ; 32-37) (Figs. 8, 9).

4. Drill guide according to claims 1 and 3, characterized in that the two radiuses (r, R) of the calotte-shaped bearing (59), the glide path (W) of the screw (16) in the hole (10) and the angle of inclination ($\alpha$) of the guideway (24) are connected by the following equation :

$$R = r + \left[ \frac{W}{2} \cdot \text{ctg} \frac{\alpha}{2} \right].$$

## Revendications

1. Calibre de perçage pour percer des trous s'étendant sous un angle d'inclinaison par rapport à l'axe longitudinal d'un os, dans des fragments d'os à stabiliser, au moyen d'un dispositif constitué d'une plaque de compression (12) avec des trous oblongs (10) ménagés dans celle-ci et de vis à tête sphérique (16 ; 32 à 37) adaptées à ces derniers et destinées à stabiliser la région d'une fracture d'os ou d'une ostéotomie dans le cas de l'ostéosynthèse à compression, caractérisé en ce qu'il comporte un canon de perçage (52, 62) monté dans un support (48, 59) en forme de calotte et destiné au guidage du foret, l'axe du canon de perçage (52, 62) étant excentré par rapport au centre (54, 0) du support (48, 59) et le support (48, 59) étant destiné à reposer, lors du perçage, dans les trous (10) de la plaque (12), tandis qu'au moins le rayon (r), situé dans un plan de coupe transversal à l'axe longitudinal (L) des trous (10), du support (48, 59) en forme de calotte est égal au rayon des têtes sphériques (17) des vis (16 ; 32 à 37).

2. Calibre de perçage selon la revendication 1, caractérisé en ce que le support en forme de calotte présente une forme sphérique (48) et est destiné à se trouver, lors du perçage, dans la voie de glissement (24) des trous (10) de la plaque (12) à l'extrémité du trou (10) voisine de la fracture, son rayon (r) étant égal au rayon des têtes sphériques (17) des vis (16 ; 32 à 37).

3. Calibre de perçage selon la revendication 1, caractérisé en ce que le rayon (r), situé dans un plan de coupe (IX-IX) transversal à l'axe longitudinal (L) du trou, du support (59) en forme de calotte est égal au rayon des têtes sphériques (17) des vis (16 ; 32 à 37), alors que le rayon (R) situé dans un plan de coupe (VIII-VIII) contenant l'axe longitudinal (L) du trou est supérieur au rayon des têtes sphériques (17) des vis (16 ; 32 à 37).

4. Calibre de perçage selon la revendication 1 ou 3, caractérisé en ce que les deux rayons (r, R) du support (59) en forme de calotte, le chemin de glissement (W) de la vis (16) dans le trou (10) et l'angle d'inclinaison ($\alpha$) de la voie de glissement (24) sont liés entre eux par l'équation suivante :

$$R = r + \left[ \frac{W}{2} \cdot \mathrm{ctg}\, \frac{\alpha}{2} \right].$$

Fig. 0

Fig. 1

Fig.2

EP 0 173 267 B1

Fig. 3

*Fig. 4*

*Fig. 5*

Fig. 6

Fig. 7

Fig. 8

IX.

0

59

67

12

62

R

IX

65

66

β = 45°

E

S

VIII

r

59

r

62

VIII

Fig. 9

Fig. 10